(19) Europäisches Patentamt
European Patent Office
Office européen des brevets

(11) EP 3 563 825 A1

# (12) EUROPEAN PATENT APPLICATION

(43) Date of publication:
**06.11.2019 Bulletin 2019/45**

(21) Application number: **18020186.5**

(22) Date of filing: **02.05.2018**

(51) Int Cl.:
*A61K 8/26* (2006.01)
*A61K 8/34* (2006.01)
*A61K 8/73* (2006.01)
*A61Q 19/10* (2006.01)
*A61P 17/10* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **The Boots Company plc**
**Nottingham NG90 1BS (GB)**

(72) Inventors:
- **Smedley, Michelle**
  **Loughborough, Leicestershire LE12 6JP (GB)**
- **Pearson, David**
  **Castle Donington, Derby DE74 2AU (GB)**
- **Diaz Fernandez, Yuri Antonio**
  **Liverpool L69 3BX (GB)**
- **Raval, Rasmita**
  **Liverpool L69 3BX (GB)**
- **Stephenson, Peter**
  **Stockport, Cheshire SK3 8AB (GB)**

(74) Representative: **Goodier, Claire-Louise**
**The Boots Company PLC**
**Group IP/Patents - D90 West F20**
**1 Thane Road West**
**Nottingham NG90 1BS (GB)**

## (54) COSMETIC SKINCARE COMPOSITION

(57) A cosmetic composition and its use in the treatment of skin conditions, such as acne vulgaris. The composition may selectively remove free fatty acids (FFAs) from a subject's skin

The composition comprises 2 to 60wt% clay; 20 to 96wt% water; and 2 to 60wt% preservative. No more than 1% carboxylic acid compounds are present and the ratio of clay to carboxylic acid compounds is at least 90wt% clay: no more than 10wt% carboxylic acid compounds.

The clay may be a 2:1 clay such as montmorillonite.

The preservative may be an alcohol such as ethanol.

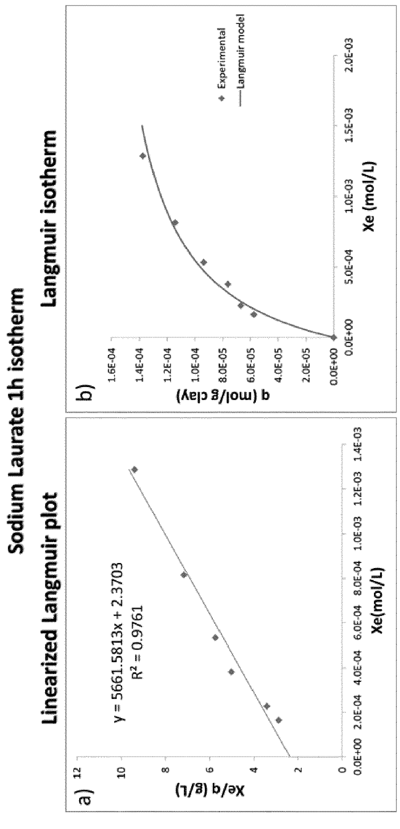


Fig 1

EP 3 563 825 A1

## Description

### Field of the invention

The present invention relates to cosmetic compositions providing improved skin protection, particularly in relation to acne, and methods of cosmetic treatment using the compositions.

### Background of the invention

Acne vulgaris is the most common skin disorder worldwide with over 100m cases in the EU and US, of which 50% require medical treatment. The disease manifests as pilosebaceous follicles in the face and upper trunk, and in severe cases can cause significant scarring and psychological problems. About 2% of UK GP appointments are related to acne and the mild-to-moderate form of acne is within the top 10 minor illnesses, costing the NHS over £2bn per year.

Despite the high occurrence of acne and its impact on quality of life and on the economy, only a few strategies have been developed to combat this skin disease and, since 1982, there have been no new approved orally administered systemic drugs for acne. The approaches currently used are based on oral medicines or topical agents that are not 100% effective and suffer from serious drawbacks. Oral medicines often have severe systemic side effects e.g. isoretinoin is linked to liver problems and birth defects. Topical and oral antibiotics increase the risk of emergence of resistant bacteria and exacerbate the antimicrobial resistance threat. Other topical agents, based on benzoyl peroxide or salicylic acid, can inflame, damage and discolour skin, and therefore their use can be detrimental. As a result there can be a low level of compliance by the patient.

### Summary of the invention

The inventors have identified a consumer need to provide cosmetic compositions which improve skin health or appearance. In particular, there is a consumer need for compositions for the treatment of skin disorders such as acne.

In a first aspect of the invention there is provided a cosmetic composition comprising:

2 to 60wt% clay;
2 to 60wt% preservative; and
20 to 96wt% water;

wherein the cosmetic composition comprises no more than 1% carboxylic acid compounds and the ratio of clay to carboxylic acid compounds is at least 90wt% clay: no more than 10wt% carboxylic acid compounds.

The inventors have determined that the use of clay in the absence of carboxylic acid compounds is beneficial for the treatment of skincare disorders including acne.

Clay is capable of adsorbing free fatty acids but has a limited number of adsorption sites. The inventors have demonstrated that carboxylic acid compounds compete for the adsorption sites, and thereby hinder the ability of the clay to adsorb free fatty acids.

Recently, the ability of Propionibacterium acnes (P. acnes) to metabolise sebum triglycerides into free fatty acids (FFAs) has been identified as an inflammatory driver of acne and the major cause of irreparable scarring (Lwin et al, Clinical and Experimental Dermatology (2014) 39, pp162-167). P. acnes bacteria exist as ubiquitous commensal organisms in healthy skin. However, on reaching certain concentrations they are believed to communicate (quorum sensing) and hydrolyse natural sebum triglycerides to produce excess FFAs acting as danger associated molecular patterns (DAMPs). The DAMPS are understood to activate receptors to express inflammatory cytokines, triggering inflammation and postulation.

The inventors propose the selective removal of inflammatory free fatty acids (FFAs) from the skin, thereby disrupting the progress of the acne vulgaris disease. This approach contrasts with traditional treatments. Many of the drugs for acne specifically target P. acnes that is believed to be implicated in the disease, when the enlarged sebaceous glands increase sebum production and become blocked. Hormone treatments are intended to reduce the production of sebum in which the P. acnes flourishes. Antibiotics are intended to reduce the population of bacteria directly and salicylic acid acts by removal of outer skin layers, re-opening the pores.

Significantly the composition of the present invention selectively removes FFAs without interfering with the normal skin bacterial flora. Skin inflamed by acne vulgaris has a higher pH than normal skin. The inventors have demonstrated that the composition of the invention is more effective at removing FFAs at higher pH when the FFAs are in the carboxylate form rather than at lower pH where the FFAs are in the acid form.

| Clay formulation | Fraction of lauric acid absorbed (acidic conditions) | Fraction of sodium laurate absorbed (basic conditions) |
|---|---|---|
| 1.5% of clay | 0.03 | 0.72 |
| 5% of clay | 0.04 | 0.96 |

The composition of the invention may have a pH of at least 6.5, at least 7, at least 7.5, at least 8, at least 8.5, at least 9 or at least 9.5 and/or no more than 10.5, no more than 10, no more than 9, no more than 8 or no more than 7.5.

The pH of healthy skin is around 5.5 whereas the pH of damaged skin is higher. The composition of the invention is more effective when its pH can respond to the pH of the skin. A pH buffer resists changes in pH. As such, the composition may not contain a pH buffer or may contain only a small quantity of buffer. The composition of the invention may have a pH of from 6.5 to 7.5 optionally in the absence of a pH buffer. The absence (or reduced amount) of a pH buffer is key in achieving selectivity for damaged skin.

A buffer solution is an aqueous solution consisting of a mixture of a weak acid and its conjugate base. Its pH changes very little when a small amount of acid or base is added to it. Citrate buffer (citric acid / sodium citrate) is a pH buffer solution commonly employed in skincare. Other commonly used buffers include Tris-HCl (a.k.a. TRIS Buffered Saline) that contains Tromethamine and Hydrochloric Acid, and Pyridoxine HC1, diglycine (gly-gly) a di-peptide that incorporates the base and acid units within the same molecular structure; HEPES 4-(2-hydroxyethyl)-1-piperazineethanesulfonic acid) used to maintain physiological pH conditions, and tricine a zwitterionic amino acid derived from TRIS and glycine.

The composition may contain no more than 3wt%, no more than 2wt%, no more than 1wt%, no more than 0.5wt%, no more than 0.2wt% or no more than 0.1wt% of a pH buffer (e.g. citrate buffer).

<u>Carboxylic acid compounds</u>

The composition comprises very little or no carboxylic acid compounds i.e. compounds that contain or generate free carboxylate such as carboxylic acids or salts thereof. Such carboxylates would compete with FFAs for adsorption on the clay.

A carboxylic acid is an organic compound that contains a carboxyl group and has the general formula R-COOH. Carboxylic acids that can be mentioned include methanoic acid, ethanoic acid (acetic acid) and citric acid (a tricarboxylic acid),

**Examples of naturally occurring carboxylic acids**

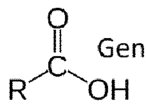
Generic structure

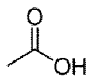
Acetic acid

lauric acid
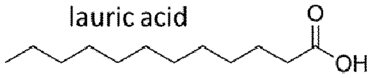

palmitic acid
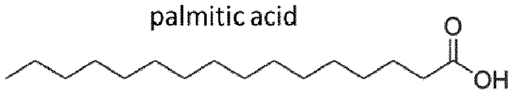

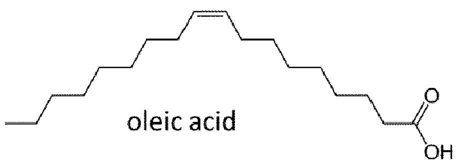
oleic acid

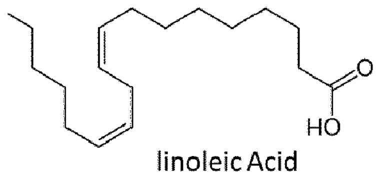
linoleic Acid

Salts of carboxylic acids are called carboxylates. Sources of free carboxylate include fatty acids (discussed below), various polysaccharides, amino acids and proteins.

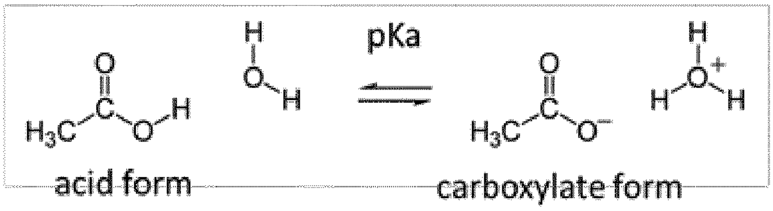

Sodium alginate is derived from alginic acid and therefore a carboxylic acid compound in the context of the present invention. Similarly gum Arabic, gellan gum and xanthan gum are derived from glucuronic acid and pectin from galacturonic acid.

Sodium carboxymethyl cellulose is a source of free carboxylate.

Acrylates (including polyacrylates) are derived from acrylic acid and are therefore considered to be a carboxylic acid compounds in the context of the present invention.

The composition may comprise no more than 0.8wt% carboxylic acid compounds, no more than 0.5wt% carboxylic acid compounds, no more than 0.3wt% carboxylic acid compounds, no more than 0.1wt% carboxylic acid compounds, no more than 0.05wt% carboxylic acid compounds or no more than 0.01wt% carboxylic acid compounds.

The ratio of clay to carboxylic acid compounds may be at least 95wt% clay: no more than 5wt% carboxylic acid compounds, at least 98wt% clay: no more than 2wt% carboxylic acid compounds, or at least 99wt% clay; no more than 1wt% carboxylic acid compounds.

Free fatty acids

A fatty acid is a carboxylic acid with a long aliphatic tail (chain), which is either saturated or unsaturated. Examples include:

| Numerical symbol | Structure $H_3C$-(R)-$CO_2H$ | Stems of systematic names | Stems of trivial names[b] | Name symbol |
|---|---|---|---|---|
| 10:0 | $-[CH_2]_8-$ | Decano- | Capr-[c] | Dec |
| 12:0 | $-[CH_2]_{10}-$ | Dodecano- | Laur- | Lau |
| 14:0 | $-[CH_2]_{12}-$ | Tetradecano- | Myrist- | Myr |
| 16:0 | $-[CH_2]_{14}-$ | Hexadecano- | Palmit- | Pam |
| 16:1 | $-[CH_2]_5CH=CH[CH_2]_7-$ | 9-Hexadeceno- | Palmitole- | ΔPam |
| 18:0 | $-[CH_2]_{16}-$ | Octadecano- | Stear- | Ste |
| 18:1(9) | $-[CH_2]_7CH=CH[CH_2]_7-$ | *cis*-9-Octadeceno- | Ole- | Ole |
| 18:1(11) | $-[CH_2]_5CH=CH[CH_2]_9-$ | 11-Octadeceno- | Vaccen- | Vac |
| 18:2(9,12) | $- [CH_2]_3(CH_2CH=CH)_2 [CH_2]_7-$ | *cis,cis*-9,12-Octadecadieno- | Linole | Lin |
| 18:3(9,12,15) | $-(CH_2CH=CH)_3[CH_2]_7-$ | 9,12,15-Octadecatrieno- | (9,12,15)-Linolen- | αLnn |
| 18:3(6,9,12) | $[CH_2]_3(CH_2CH=CH)_3 [CH_2]_4-$ | 6,9,12-Octadecatrieno- | (6,9,12)-Linolen- | γLnn |
| 18:3(9,11,13) | $-[CH_2]_3(CH=CH)_3[CH_2]_7-$ | 9,11,13-Octadecatrieno- | Eleostear- | eSte |

The dissociation equilibrium of fatty acids can be described by the acidity constant, often expressed in logarithmic units as pKa. At pH values below the pKa (more acidic), the fatty acid molecules are predominantly in the protonated acid form, while for pH values above the pKa (more basic), the carboxylic group is deprotonated to form charged carboxylate species. For medium and long-chain fatty acids, the pKa is nearly constant when the length of the hydrocarbon chain increases. In ethanol-water mixtures, the pKa of fatty acids is generally higher with respect to the pKa values in pure water, but for mixtures up to 60% in weight of ethanol the pKa values indicate that the fatty acids are deprotonated at pH=6 or above, irrespective of the presence of ethanol in the solvent mixture.

Clay

The composition comprises clay to selectively adsorb FFAs.

Adsorption is the adhesion of atoms, ions or molecules from a gas, liquid or dissolved solid to a surface. This process differs from absorption, in which a fluid is dissolved by or permeates a liquid or solid, respectively. Adsorption is a surface-based process while absorption involves the whole volume of the material. However, in the case of highly porous materials, the distinction is more semantic than physical.

There are three main classes of clay: kaolinite, smectite and illite.

Clays are classified as either 1:1 or 2:1 referring to the structure of the silicate sheets which make up the mineral. 1:1 clays have one tetrahedral sheet and one octahedral sheet and examples include kaolinite and serpentine. 2:1 clays have one octahedral sheet between two tetrahedral sheets and examples include montmorillonite, vermiculite and talc. Most 2:1 clays are expansive clays, which swell and shrink with water content.

The smectite clays are known as "expansive" clays. Montmorillonite has the highest swell-shrink behaviour of the smectite clays.

Illite clays are considered non expansive.

The composition may comprise at least 3wt%, at least 4wt%, at least 5wt%, at least 6wt%, at least 8wt%, at least 10wt%, at least 15wt%, at least 20wt%, at least 30wt%, at least 35wt%, at least 40wt%, at least 45wt% or at least 50wt% clay and/or the clay may comprise no more than 55wt%, no more than 52wt%, no more than 50wt%, no more than 45wt%, no more than 40wt%, no more than 35wt%, no more than 30wt%, no more than 25wt%, no more than 20wt%, no more than 15wt%, or no more than 10wt%.

The clay has a high adsorption capacity that may allow the use of low clay concentrations, especially for the relatively low concentrations of FFA expected on the skin. in vitro experiments have demonstrated that 1g of clay can uptake up to 39mg of lauric acid.

A composition comprising 5wt% clay was effective for removing a least a fraction of 0.96 of lauric acid, under in vitro conditions.

The composition may comprise from 3 to 10wt% clay, e.g. 4 to 8wt% clay (e.g. montmorillonite). Such compositions have been shown to be useful as liquid cosmetic compositions.

The composition may comprise from 15 to 35wt% clay, such as 20 to 30wt% clay (e.g. montmorillonite). Such compositions have been shown to be useful as gel cosmetic compositions, e.g. a face mask.

Preservative

A preservative is a substance that is included in the composition to prevent decomposition, e.g. due to microbial growth. The composition comprises one or more preservatives.

Preservatives that can be mentioned include 2-bromo-2nitropropane-1,3-diol (bronopol, commercially available under the trade name Myacide RTM), benzyl alcohol, diazolidinyl urea, imidazolidinyl urea, methyl paraben, phenoxyethanol, ethyl paraben, propyl paraben, isothiazolone, and/or chlorphensin. In one embodiment, the composition of the invention does not comprise parabens.

The preservative may consist of or comprise alcohol i.e. an organic compound in which the hydroxyl functional group (-OH) is bound to a saturated carbon atom. This has a particular benefit in providing a co-solvent as well as preservative properties.

The preservative may comprise an alcohol in combination with another preservative, e.g. alcohol (such as ethanol) together with another preservative selected from 2-bromo-2nitropropane-1,3-diol, diazolidinyl urea, imidazolidinyl urea, methyl paraben, phenoxyethanol, ethyl paraben, propyl paraben, isothiazolone, and/or chlorphensin.

The preservative may comprise a short-chain alcohol (1 to 3 carbons), a medium-chain alcohol (4 to 7 carbons), a long chain alcohol (8 to 21 carbons) or a very long chain alcohol (22 carbons or more).

The preservative may comprise or consist of an aliphatic alcohol.

The preservative may comprise or consist of a primary, secondary or tertiary alcohol.

The preservative may comprise or consist of a monohydric alcohol, e.g. a monohydric aliphatic alcohol.

Alcohols that can be mentioned include methanol, ethanol, propanol, butanol, pentanol, lauryl alcohol, cetyl alcohol, octyldodecanol, stearyl alcohol, behenyl alcohol, myristyl alcohol, oleyl alcohol, cetostearyl alcohol, monostearyl glycerine ether (batyl alcohol), 2-decyltetradecinol, lanolin alcohol, cholesterol, phytosterol, hexyldodecanol, isostearyl alcohol, and benzyl alcohol.

In embodiments the preservative comprises methanol, ethanol and/or propanol.

In embodiments the preservative comprises or consists of ethanol.

The preservative (e.g. ethanol) may be present in an amount of about 3wt% to about 50wt% of the composition, about 5wt% to about 40wt% of the composition, about 10wt% to about 35wt% of the composition or about 20% to about 30% by weight of the composition. The preservative (e.g. ethanol) may be present in an amount of 20wt% to 50wt%.

Formulations

The cosmetic composition may comprise (or consist of):

3 to 50wt% (e.g. 5 to 40wt%) clay;
3 to 50wt% (e.g. 5 to 40wt%) preservative; and
20 to 94wt% water

wherein the cosmetic composition comprises no more than 1wt% (e.g. no more than 0.5wt%) carboxylic acid compounds and the ratio of clay to carboxylic acid compounds is at least 90wt% (e.g. at least 95wt%) clay: no more than 10wt% (no more than 5wt%) carboxylic acid compounds.

The cosmetic composition of the first aspect may comprise 3 to 15wt% clay; 20 to 40wt% preservative; and 45 to 77wt% water.

The cosmetic composition of the first aspect may comprise 4 to 10wt% clay; 25 to 35wt% preservative; and 55 to 71wt% water.

The cosmetic composition of the first aspect may comprise 15 to 40wt% clay; 20 to 40wt% preservative; and 20 to 45wt% water.

The cosmetic composition of the first aspect may comprise 15 to 30wt% clay; 25 to 35wt% preservative; and 35 to 60wt% water.

Other ingredients

The composition may consist of clay (e.g. montmorillonite), water and preservative (e.g. alcohol). Alternatively it may contain additional ingredients.

The composition may comprise a thickener. This allows the viscosity to be modified if desired. The thickener may not comprise a carboxylic acid group.

The composition may comprise at least 0.1wt%, at least 0.2wt%, at least 0.5wt%, at least 1wt%, at least 2wt%, at least 3wt% or at least 5wt% thickener and/or no more than 10wt%, no more than 6wt%, no more than 4w%, no more than 2wt% or no more than 1wt% thickener.

Suitable thickeners include cellulose based thickeners such as cellulose itself, methyl cellulose, and hydroxyalkyl cellulose, such as hydoxyethyl cellulose, and hydroxyl propyl cellulose. Carboxymethyl cellulose is not preferred due to its carboxy group.

The composition may comprise from 0.1 to 5wt% hydroxyalkyl cellulose. Such compositions were found to be successful in the examples, having an appropriate viscosity for cosmetic and personal care products.

The composition may comprise at least 3wt% hydroxylethyl cellulose and/or at least 3wt% hydroxypropyl cellulose.

The composition may comprise no more than 5wt%, no more than 3wt%, no more than 1wt% or no more than 0.5wt% Magnesium Aluminium Silicate. Such components may interfere with composition's ability to adsorb FFAs selectively.

The composition may comprise no more than 5wt%, no more than 3wt%, no more than 1wt% or no more than 0.5wt% carrageenan. We submit that carrageenan might generate free sulphates and/or cause electrostatic interference.

Polyphenols may be avoided. The composition may comprise no more than 5wt%, 3wt% or 1wt% polyphenols. Many polyphenols are known to be acidic (e.g. tannic acid), forming anionic species at basic pH. These anionic species may compete with FFA for adsorption sites on the clay.

Applications

The cosmetic composition of the present invention may be a face mask. The composition may be applied to the skin for at least 5 minutes and no more than one hour.

The cosmetic composition of the present invention may be a cleanser. The composition may be applied to the skin for at least 2 minutes and no more than 10 minutes.

Packaging

The composition may be provided in a sealed container, such as a water-tight container. The container may be tub, a tube or a sachet for example.

The container may have a volume of at least $5cm^3$, $10cm^3$, $20cm^3$, $50cm^3$, $100cm^3$, or $200cm^3$ and/or no more than $500cm^3$, $300cm^3$, $200cm^3$, $100cm^3$, or $50cm^3$.

The container may be a single-use container (e.g. sachet) or a multiple use container that is resealable.

According to a second aspect of the invention there is provided a method for the preparation of the composition of the first aspect, the method comprising mixing clay, preservative and water to yield a homogenous mixture.

The mixing may take place at standard ambient temperature and pressure (SATP, 25°C, 100kPa).

The mixing may be carried out using an overhead stirrer or a homogenizer.

In one embodiment the clay (e.g. montmorillonite) and the preservative (e.g. alcohol) are mixed with an overhead stirrer, and then water (e.g. deionised water) is added to achieve the required volume.

In one embodiment the preservative (e.g. alcohol) and at least a portion of the water is mixed, and then the clay is added and mixed to achieve the homogenous mixture. Further water may be added to achieve the required volume.

According to a third aspect of the invention there is provided a method of cosmetic treatment of a skin condition comprising the step of applying the cosmetic composition of the first aspect onto the skin of a subject afflicted with the skin condition or at risk of being afflicted with the skin condition.

The cosmetic composition may be applied to the skin by hand or using an applicator (e.g. cotton wool). The composition may be applied to the skin for at least 5 minutes, at least 10 minutes, or at least 20 minutes and/or no more than two hours, one hour, 30 minutes or 20 minutes.

The skin condition may be a skin disorder such as acne vulgaris.

According to a fourth aspect of the invention there is provided a use of the cosmetic composition according to the first aspect for selective removal of free fatty acids (FFAs) from the skin.

As explained above, the composition selectively removes FFAs in the carboxylate form, in preference to the acid form. This allows the selective removal of FFAs from damaged skin (e.g. the surface of the skin), in preference to healthy skin.

According to a fifth aspect of the invention there is provided the composition of the first aspect for use as a medicament.

The composition of the first aspect may be applied to the skin (topical application), e.g. to the face, as a medical treatment.

According to a sixth aspect of the invention there is provided the composition of the first aspect for use in the treatment of skin disorders, such as acne vulgaris.

The composition of the first aspect may be applied to the skin (topical application), e.g. to the face, in order to treat a skin disorder, such as acne vulgaris.

Embodiments of the invention will now be described with reference to the following figures:

Figure 1: isotherm data for sodium laurate (37°C, 40% ethanol 60% water, 1h) showing: a) the linearized plot used to obtain the isotherm characteristic parameters, the best fitting line equation is shown in the inset; b) combined experimental and model fitting for the isotherm; and

Figure 2 a bar chart showing the reduction of free fatty acids achieved by compositions in accordance with embodiments of the invention.

**METHODOLOGY**

The table lists the typical physical properties of one montmorillonite (Sigma Aldrich, CAS Number: 1318-93-0).

| | |
|---|---|
| Surface Area($m^2$/g) | 220-270 |
| Loss on ignition (%) | 7 |
| Bulk density(g/l) | 300-370 |
| nm range | pore volume (ml/g by $CCl_4$) |
| 0-80 | 0.318 |
| nm range | pore volume (ml/g by Hg porosimeter) |
| 15000-1750 | 0.29 |
| 1750-80 | 0.53 |
| 80-14 | 0.13 |
| 14-7.5 | 0.07 |

**Face mask "gel" product containing montmorillonite**

| Ingredient (% w/v) | Ex. 1 | Ex. 2 | Ex. 3 | Ex. 4 |
|---|---|---|---|---|
| Montmorillonite | 20 | 27 | 30 | 27 |
| Ethanol | 30 | 30 | 30 | 30 |
| Glycerol | 0 | 0 | 0 | 5 |
| Propylene glycol | 0 | 0 | 0 | 3 |
| Water | q.s. | q.s. | q.s. | q.s. |
| Comment | Viscous homogenous gel with evidence of a | Viscous homogenous gel with evidence of a | Viscous homogenous gel with evidence of a | Viscous homogenous gel with evidence of a |
| | slight shiny texture | matte texture | matte texture | slight shiny texture |

Examples 1 to 4 are intended as a "wash-off" system that can be applied to all skin types, e.g. applied on the face and left on the skin for 5 to 30 minutes. The following ingredients were avoided:

- Carboxylic acids and carboxylic acid salts
- Amino acids
- pH buffers and stabilizers
- polyphenols
- oils containing fatty acids
- natural products, perfumes, colours and additives

The level of ethanol chosen was 30% w/w. Since ethanol has preservative properties, no microbial testing is required.

Ex.1 was the preferred formulation. It was easily prepared and a viscous homogenous gel was formed. Ex. 3 was more difficult to prepare since the viscosity was higher.

The presence of humectants (Ex. 4) did not provide great value to the appearance of the gel (c.f. Ex. 2) so it was decided to investigate formulations containing only water, ethanol and the clay.

**Stability testing**

Samples were prepared using the following method:

1. Dispense the organic solvent (ethanol) into a suitable vessel.

2. Dispense 80% of the de-ionised water into the vessel and mix using an overhead stirrer.

3. Dispense the clay and slowly add to the vessel. Mix until homogenous with an overhead stirrer.

4. Make up to volume with de-ionised water and further mix for 5 minutes.

Three batches (examples 5 to 7) were prepared for stability testing and dispensed into 60ml clear glass jars with black lids.

| Ingredient (% w/v) | Ex. 5 | Ex. 6 | Ex. 7 |
|---|---|---|---|
| Montmorillonite | 15 | 20 | 25 |
| Ethanol | 30 | 30 | 30 |
| Water | q.s | q.s. | q.s. |

Samples were stored at 25°C/60% RH (relative humidity), 30°C/65% RH and 40°C/75% RH for T = 1 months and T = 3 months times points. At each time point the samples were tested for appearance; pH; specific gravity (tested using a small glass vial); and viscosity (Viscometer RV model with helipath stand at 5 rpm; spindle TC Ex. 5, spindle TD Ex. 6 and spindle TF Ex. 7).

**Example 5**

| Time point | Condition | Appearance | Specific Gravity | pH | Viscosity (cP '000) |
|---|---|---|---|---|---|
| Initial | N/A | Light brown shiny viscous homogenous gel. | 1.02 | 9.4 | 94 |
| 1 Month | 25°C 60% RH | Soft shiny beige coloured clay mask. No evidence of phase separation. | 1.05 | 9.1 | 100 |
| | 30°C 65% RH | | 1.05 | 9.1 | 130 |
| | 40°C 75% RH | | 1.05 | 9.1 | 155 |
| 3 Month | 25°C 60% RH | Soft shiny beige coloured clay mask. No evidence of phase separation. | 1.04 | 9.0 | 130 |
| | 30°C 65% RH | Soft shiny beige coloured clay mask. No evidence of phase separation. Slight evidence of dryness. | 1.03 | 9.1 | 180 |
| | 40°C 75% RH | Soft matte beige colour face mask. Slight evidence of dryness. No evidence of phase separation. | 1.04 | 9.0 | 200 |

**Example 6**

| Time point | Condition | Appearance | Specific Gravity | pH | Viscosity (cP '000) |
|---|---|---|---|---|---|
| Initial | N/A | Light brown slightly shiny viscous homogenous gel. | 1.09 | 9.2 | 240 |
| 1 Month | 25°C 60% RH | Soft matte beige clay mask. No evidence of phase separation. | 1.08 | 9.1 | 455 |
| | 30°C 65% RH | | 1.08 | 9.1 | 760 |
| | 40°C 75% RH | | 1.08 | 9.0 | 945 |
| 3 | 25°C | Soft slightly shiny beige colour face | 1.07 | 9.1 | 600 |
| Month | 60% RH | mask. No evidence of phase separation. | | | |
| | 30°C 65% RH | Soft slightly shiny beige colour face mask. Evidence of dryness present. No evidence of phase separation. | 1.07 | 9.0 | 950 |
| | 40°C 75% RH | Soft matte beige colour face mask. Slight evidence of dryness. No evidence of phase separation. | 1.08 | 9.0 | 1200 |

**Example 7**

| Time point | Condition | Appearance | Specific Gravity | pH | Viscosity (cP ’000) |
|---|---|---|---|---|---|
| Initial | N/A | Light brown viscous matte homogenous gel. | 1.10 | 9.0 | 940 |
| 1 Month | 25°C 60% RH | Soft matte beige clay mask. No evidence of phase separation, however slight evidence of dryness. | 1.09 | 9.2 | 8100 |
| | 30°C 65% RH | | 1.10 | 9.1 | 10800 |
| | 40°C 75% RH | | 1.11 | 9.0 | 15200 |
| 3 Month | 25°C 60% RH | Soft matte beige colour face mask. No evidence of phase separation. Evidence of dryness present. | 1.09 | 9.1 | 9000 |
| | 30°C 65% RH | | 1.09 | 9.1 | 11400 |
| | 40°C 75% RH | | 1.09 | 9.1 | 17800 |

Overall the results were consistent throughout the three month stability testing. In particular, all formulations had a pH of 9.0 to 9.4 over the course of testing and consistent specific gravity.

**Face mask "liquid" product containing montmorillonite**

This product is intended to have a viscosity similar to a toner and the concept is a "wash off" system that can be applied to all skin types, e.g. by cotton wool, and left for 5 to 30 minutes.

As above, the following ingredients were avoided:

- carboxylic acids and carboxylic acid salts
- Amino acids
- pH buffers and stabilizers
- polyphenols
- oils containing fatty acids
- natural products, perfumes, colours and additives

The level of ethanol chosen was 30% w/w. Since ethanol has preservative properties, no microbial testing is required.

| Ingredient (% w/v) | Ex. 8 | Ex. 9 | Ex. 10 | Ex.11 | Ex. 12 | Ex. 13 | Ex. 14 | Ex. 15 |
|---|---|---|---|---|---|---|---|---|
| Montmorillonite | 4 | 4 | 4 | 4 | 4 | 4 | 4 | 4 |
| Ethanol | 30 | 30 | 30 | 30 | 30 | 30 | 30 | 30 |
| Klucel E CS (hydroxypropyl cellulose) | 0 | 0.3 | 0.5 | 0.8 | 0 | 0 | 0 | 0 |
| Natrosol 250 HX (hydroxyethyl cellulose) | 0 | 0 | 0 | 0 | 0.3 | 0.5 | 0.8 | 0.9 |
| Water | q.s. | q.s. | q.s. | q.s. | q.s. | q.s. | q.s. | q.s. |

Ex. 8 contains 4% montmorillonite in ethanol/water. Over a short period, there was evidence of sedimentation so thickeners were investigated.

Examples 9 to 11 employ hydroxypropyl cellulose at different levels. Homogenous, slightly viscous suspensions were formed. There was some sedimentation overnight but this was easily re-dispersed after two or three inversions of the glass jar.

Examples 12 to 15 employ hydroxyethyl cellulose at different levels. After overnight storage there was no evidence of sedimentation.

Further formulations were then developed based on 8% (w/w) clay.

| Ingredient (% w/v) | Ex. 16 | Ex. 17 | Ex. 18 | Ex. 19 |
|---|---|---|---|---|
| Montmorillonite | 8 | 8 | 8 | 8 |
| Ethanol | 30 | 30 | 30 | 30 |
| Klucel E CS (hydroxypropyl cellulose) | 0 | 0 | 0 | 2 |
| Natrosol 250 HX (hydroxyethyl cellulose) | 0.414 | 0.2 | 0.15 | 0.1 |
| Water | q.s. | q.s. | q.s. | q.s. |

Ex. 16 contained less hydroxyethyl cellulose than Ex. 13 to 15. There was evidence of gel formation after overnight storage at room temperature.

Ex. 17 was prepared by mixing the clay using an overhead stirrer. A slightly viscous homogenous suspension was formed. There was some evidence of sedimentation after overnight storage.

Ex. 18 was prepared using a homogeniser (Silverson) during mixing of the clay. This formed a gelatinous mass after overnight storage at room temperature.

Ex. 19 contained both hydroxypropyl cellulose and hydroxyethyl cellulose. After overnight storage there was evidence of caking.

Comparing Ex. 17 and Ex. 18, it was seen that homogenisation had a great effect on the viscosity of the final product. Therefore a short study was performed to compare the mixing methods.

| Ingredient (% w/v) | Ex. 20 | Ex. 21 | Ex. 22 |
|---|---|---|---|
| Montmorillonite | 8 | 8 | 8 |
| Ethanol | 30 | 30 | 30 |
| Klucel E CS (hydroxypropyl cellulose) | 3 | 3 | 4 |
| Water | q.s. | q.s. | q.s. |
| Comment | Homogenised | Overhead stirrer | Overhead stirrer |

Ex. 20 and Ex, 21 were identical except that Ex. 20 was homogenised using a Silverson whereas Ex. 21 was prepared using an overhead stirrer. It was evident that Ex. 20 was more viscous that Ex. 21. since this formed a viscous homogenous suspension whereas Ex. 21 showed evidence of sedimentation.

Ex, 22 was prepared by mixing using an overhead stirrer containing a higher level of thickener. Slight evidence sedimentation was evident after overnight storage at room temperature.

In summary, for examples 8 to 17, the clay was incorporated by mixing with an overhead stirrer. However, it was noticed that the montmorillonite has similar properties to a thickening agent when homogenised using a Silverson. Therefore the process was altered.

Since the clay hydrolyses better in water, the level of ethanol was reduced to 26% w/v.

| Ingredient (% w/v) | Ex. 23 | Ex. 24 | Ex. 25 | Ex. 26 | Ex. 27 | Ex. 28 | Ex. 29 |
|---|---|---|---|---|---|---|---|
| Montmorillonite | 4 | 6 | 8 | 4 | 5 | 4 | 4 |
| Ethanol | 26 | 26 | 26 | 30 | 26 | 26 | 26 |
| Klucel E CS (hydroxypropyl cellulose) | 0 | 0 | 0 | 0 | 0 | 2 | 3 |
| Water | q.s. | q.s. | q.s. | q.s. | q.s. | q.s. | q.s. |
| Comment | A | B | B | A | A | A | A |
| A = Slightly viscous homogenous suspension<br>B = Viscous homogenous gelatinous mass | | | | | | | |

Examples 23 to 27 were prepared without thickening agents and stored at 25°C / 60% RH and 50°C in order to assess the sedimentation of the clay.

It was noticed that examples 23, 26 and 27 showed evidence of sedimentation after T = 7 days whereas

examples 24 and 25 formed a gelatinous mass. Also with the reduction of the ethanol level the viscosity of the suspension increased as demonstrated Ex. 23 being visually more viscous than Ex. 26.

Examples 28 and 29 were prepared with thickener to avoid sedimentation. There was slight evidence of phase separation for Ex. 28, however Ex. 29 was a homogenous suspension.

**Stability testing**

| Ingredient (% w/v) | Ex. 30 |
|---|---|
| Montmorillonite | 4 |
| Ethanol | 26 |
| Klucel E CS (hydroxypropyl cellulose) | 3 |
| Water | q.s. |
| Comment | A |

Ex. 30 was prepared for stability testing and dispensed into 60ml clear glass jars with black lids using the following method:

1. Dispense the ethanol into a suitable vessel.
2. Dispense the hydroxypropyl cellulose and add to the vessel. Mix until a clear solution appears using an overhead stirrer.
3. Dispense the montmorillonite clay and slowly add to the vessel. Mix until homogenous using an overhead stirrer.
4. Make up to volume with de-ionised water and mix using a homogeniser (Silverson).

| **Time point** | **Condition** | **Appearance** | **Specific Gravity** | **pH** | **Viscosity (cP)** |
|---|---|---|---|---|---|
| Initial | N/A | Slightly viscous homogenous suspension. | 0.99 | 9.4 | 270 |
| 1 Month | 25°C 60% RH | Evidence of the clay sedimenting at the bottom of the bottle. Easily re-dispersed once shaken for few seconds. | 0.99 | 9.1 | 100 |
| | 30°C 65% RH | | 0.99 | 9.1 | 119 |
| | 40°C 75% RH | | 0.98 | 9.2 | 107 |
| 2 Month | 25°C 60% RH | Evidence of the clay sedimenting at the bottom of the bottle. Easily re-dispersed once shaken for few seconds. | 0.98 | 9.2 | 118 |
| | 30°C 65% RH | | 0.98 | 9.2 | 118 |
| | 40°C 75% RH | | 0.98 | 9.4 | 112 |
| 3 Month | 25°C 60% RH | Evidence of the clay sedimenting at the bottom of the bottle. Easily re-dispersed once shaken for few seconds. | 0.99 | 9.1 | 105 |
| | 30°C 65% RH | | 0.99 | 9.3 | 113 |
| | 40°C 75% RH | | 0.99 | 9.4 | 110 |

**Experimental details.**

**Acid number test**

The amount of FFA acid present in solution was determined initially using the established Acid Number Test, with phenolphthalein as an indicator of the titration end point. All the titrations were performed in a mixture of 40% ethanol and 60% water (% in weight) prepared using neutralised ethanol. In a typical experiment, 5ml of sample were diluted with 20ml of solvent mixture in a conical flask, adding then 3 droplets of phenolphthalein indicator. Standard sodium hydroxide solution (4 $10^{-3}$M, diluted from 1M Sigma Aldrich standard) was used as titre solution. The end point of the titration was determined visually, by the change in colour of the solution. A set of 3 consistent replicates were performed

for each titration point.

**pH-metric titrations**

For the analysis of the FFA in the deprotonated carboxylate form, we implemented a pH-metric titration method using a HI-1131B glass electrode connected to a HI-5522 Hanna Instruments pH-meter. All the titrations were performed in a mixture of 40% ethanol and 60% water (% in weight) prepared using neutralised ethanol, unless otherwise indicated. In a typical experiment, 5ml of sample were diluted with 20ml of solvent mixture in a beaker containing a magnetic stirrer. The electrode was carefully immersed in the solution that was then kept under constant stirring using a magnetic plate. This solution was titrated using nitric acid (4 $10^{-3}$M) until pH=4 was reached. At least 3 consistent replicates were performed for each titration point.

Before each set of experiments, a control sample with known concentration of FFA was analysed, allowing the determination of the moles of FFA in the unknown samples. This method was used to evaluate the absorption of FFA on the clay, following the protocol described in the next subsection. In this case, the moles of absorbed FFA were determined by difference, correcting the data for volume dilutions, electrode response, and residual acidity of the clay. Similarly, for the determination of FFA in the protonated acidic form, we adapted the potentiometric method, using the reversed procedure (titration with base).

**Fatty acids absorption isotherm: the Langmuir model**

The absorption of FFA on different clays has been studied previously using the physical model developed by Irving Langmuir. This model (a.k.a. Langmuir isotherm) considers that the solid absorbent material (clay) has specific active sites on which the solute molecules (FFA) can be reversibly absorbed from the solution. The equilibrium between free and absorbed FFA can be described by the expression:

$$\frac{q}{q_{max}} = \frac{K_L \cdot C_{FFA}}{1 + K_L \cdot C_{FFA}}$$

Eq.1

Where $C_{FFA}$ is the concentration of FFA in solution, $K_L$ is the absorption equilibrium constant, $q$ is the amount of FFA absorbed per gram of absorbent material, and $q_{max}$ is the amount of FFA that saturates the absorbent. This expression can be also written in a linear form that allows the determination of the parameters $K_L$ and $q_{max}$ using the experimental data to plot ($C_{FFA}$/q) vs $C_{FFA}$:

$$\frac{C_{FFA}}{q} = \frac{C_{FFA}}{q_{max}} + \frac{1}{K_L \cdot q_{max}}$$

Eq.2

The absorption equilibrium, determined by the constant $K_L$ and the maximum amount of FFA absorbed by the clay $q_{max}$, depends on the dispersion solvent used, on the pH of the solution, and on the molecular structure and chemical form of the FFA.

The absorption of FFA on the selected clay montmorillonite K10 was investigated using solutions of the fatty acid salts in a solvent mixture 40% ethanol and 60% water. The clay Montmorillonite K10 was purchased from Sigma Aldrich and was used without any further treatment, unless otherwise specified. Three model fatty acid salts were investigated (sodium laurate, sodium oleate, sodium palmitate, Sigma Aldrich) covering saturated and unsaturated FFA of different chain lengths.

For the determination of the Langmuir isotherm parameters we adapted a method previously reported in the literature. Exact amounts of clay (from 0.1g to 0.6g) were placed in 50ml falcon tubes and 20ml of the fatty acid salt solutions (2 x $10^{-3}$M, solvent mixture 40% ethanol and 60% water) were added, stirring vigorously to lead a cloudy suspension. The samples were then immediately placed in a thermal bath at 37°C and equilibrated for 45min. After this equilibration time, the samples were centrifuged for 15min in a thermostated pre-heated centrifuge (T=37°C) to sediment the suspended clay. The overall equilibration time at 37°C for each isotherm experiment was 1h. After centrifugation, aliquots of 5ml were immediately taken from the supernatant on each sample to perform the potentiometric titration method described above. All the experiments were carried out in triplicates.

Linearized plots and the isotherms were obtained for three model fatty acids: sodium laurate, sodium oleate and sodium palmitate. The isotherm data for sodium laurate is shown in figure 1 for the purpose of illustration. The plots showed that the Langmuir model describes accurately the experimental data for the absorption of FFA at 37°C on montmorillonite K10. Using the linear plots we were able to determine the isotherm parameters for the three fatty acids studied here. From Eq.2, the expressions for $K_L$ and $q_{max}$ can be written:

$$q_{max} = \frac{1}{m} \ ; \ K_L = \frac{m}{n}$$

Eq.3

| | $q_{max}$ (mol/g of clay) | $q_{max}$ (mg/g of clay) | $K_L$ (L/mol) | $K_L$ (L/mg) |
|---|---|---|---|---|
| laurate | $1.76\ 10^{-4}$ | 39.2 | 2388.55 | $1.07\ 10^{-2}$ |
| oleate | $2.03\ 10^{-4}$ | 45.1 | 2080.72 | $9.36\ 10^{-3}$ |
| palmitate | $2.38\ 10^{-4}$ | 66.4 | 1104.20 | $3.97\ 10^{-3}$ |

The parameters indicate that the adsorption capacity of the clay is 40-70mg of FFA per each gram of absorbent clay. We also observed relatively high equilibrium constants indicating that the absorption of the FFA on the clay is thermodynamically favourable. These parameters can be used to estimate the fraction of FFA absorbed by the clay in the dilute regime, where the concentration of FFA is low and the amount of absorbent clay is high, as expected in the working conditions for the final application, where the formulation will be in contact with the inflamed skin. Under these conditions, we can estimate that more than 98% of FFA will be absorbed after 1h for formulations containing at least 20% of clay or higher clay loads in 40% ethanol / 60% water mixture.

**Residual acidity of the absorbent material**

During the FFA absorption isotherm experiments discussed above, we systematically observed that the clay had an effect on the pH of the solutions. For high concentrations of clay, the pH was progressively lower, reaching values below pH=4. We performed release experiments in the presence of different loads of clay in 40% ethanol / 60% water, to evaluate the amount of acid released by the clay into the solvent mixture.

The intrinsic acidity of the clay was evaluated using a procedure similar to the isotherm protocol, but in the absence of fatty acid salts in the solvent mixture. The residual amount of acid released from clay was determined by potentiometric titration, using sodium hydroxide ($4\ 10^{-3}$ M, diluted from 1M Sigma Aldrich standard). These experiments were also carried out using the Acid Number test protocol, but the potentiometric method was more reproducible and therefore was used widely. The residual acidity of the clay was expressed in moles of $H^+$/g of clay.

| g clay | moles $H^+$ released | % error of FFA salt in 20ml |
|---|---|---|
| 0.1 | $4.62\ 10^{-07}$ | 1.15 |
| 0.2 | $9.24\ 10^{-07}$ | 2.31 |
| 0.3 | $1.53\ 10^{-06}$ | 3.81 |
| 0.4 | $1.68\ 10^{-06}$ | 4.21 |
| 0.5 | $1.93\ 10^{-06}$ | 4.82 |
| 0.6 | $2.31\ 10^{-06}$ | 5.79 |

Following potentiometric titrations of the supernatant solutions, after 1h of contact with the clay at 37°C, we determined an average release of 4.37 μmole of H+/g of clay. This value introduced a systematic error below 6% in the determination of the FFA salts absorbed for all the amounts of clay used here. This error was corrected for the calculation of the isotherms, considering that the acid released from the clay would already neutralised a proportional fraction of the FFA salts initially present in the solution. The entire isotherm data set presented above was corrected by this factor, and therefore the final data discussed is independent of the acidity of clay. As expected, the correction was more important

for higher amounts of clay, while for small clay loads the correction was negligible. Nevertheless, it is worth considering that the clay itself changes the pH of the solution.

**Preferential absorption of protonated FFA vs FFA salts**

The absorption of FFA on the selected clay montmorillonite K10 was investigated using solutions of the fatty acid salts in a solvent mixture 40% ethanol and 60% water. The clay Montmorillonite K10 was purchased from Sigma Aldrich and was used without any further treatment, unless otherwise specified. One model fatty acid and one model fatty acid salt were investigated (i.e. lauric acid and sodium laurate, Sigma Aldrich). For the determination of the adsorption, 1g (or 0.3g) of clay samples were placed in 50ml falcon tubes and 20ml of the fatty acid or fatty salt solutions (2 x 10-3M, solvent mixture 40% ethanol and 60% water) were added, stirring vigorously to lead a cloudy suspension. The samples were then immediately placed in a thermal bath at 37°C and equilibrated for 45min. After this equilibration time, the samples were centrifuged for 15min in a thermostated pre-heated centrifuge (T=37°C) to sediment the suspended clay. The overall equilibration time at 37°C for each isotherm experiment was 1h. After centrifugation, aliquots of 5ml were immediately taken from the supernatant on each sample to perform the potentiometric titration method described above. All the experiments were carried out in triplicates.

| Clay formulation | Fraction of lauric acid absorbed (acidic conditions) | Fraction of sodium laurate absorbed (basic conditions) |
|---|---|---|
| 1.5% of clay | 0.03 | 0.72 |
| 5% of clay | 0.04 | 0.96 |

These results suggest that while the formulation is able to remove over 96% of the FFAs under basic pH conditions and with clay concentrations as low as 5% of clay in the formulation, the system is unable to remove the FFAs in the acidic form, under acidic pH conditions resembling healthy skin. These data demonstrate that our formulation is responsive to pH changes, being able to facilitate the absorption of FFAs in basic pH conditions associated with inflamed and damaged skin.

**Fixed mass isotherm experiments**

The adsorption of FFA on the selected clay montmorillonite K10 was investigated using different solvent conditions. The clay Montmorillonite K10 was purchased from Sigma Aldrich and was used without any further treatment, unless otherwise specified. Two model fatty acid salts were investigated (i.e. sodium laurate and sodium oleate, Sigma Aldrich). For the determination of the adsorption, 0.6g of clay samples were placed in 50ml falcon tubes and 20ml of the fatty salt solutions (2 x $10^{-3}$M) in different solvent mixtures from 55% to 0% ethanol in water were added, stirring vigorously to lead a cloudy suspension. Two equilibration times were investigated, 60 min and 5min. For the 60min, the samples were equilibrated for 45 min in a thermostat bath, and then subsequently centrifuged in a thermostated pre-heated centrifuge (T=37°C) for 15min to sediment the suspended clay. For the 5min experiments, the samples were immediately centrifuged for 5min in a thermostated pre-heated centrifuge (T=37°C) to sediment the suspended clay. After centrifugation, aliquots of 5ml were immediately taken from the supernatant on each sample to perform the potentiometric titration method described above. All the experiments were carried out in triplicate.

These experiments were carried out using a formulation containing 3% of clay over a period of time compatible with a cleansing format (5 min) or leave on product (60min). The resulting adsorption data for the model FFA salts (sodium laurate and sodium oleate) demonstrated that solvent composition has little effect on the FFA adsorption performance of the formulation.

The amount of FFA salt adsorbed by the clay changed less than 2% going from pure water to 40% ethanol. This behaviour can be explained considering that water is a good polar solvent for deprotonated carboxylic groups. Therefore, even at relatively high ethanol compositions, the hydration thermodynamics is dominated by water. Additionally, this data confirms that the mechanism of adsorption of the FFA on the clay is related to specific interactions of the charged carboxylate species with the clay surface. This data is consistent with previous observations of the pH responsivity of the system that is also regulated by specific interactions of the carboxylic group.

Comparison of solvent composition effect on adsorption of FFA salts (3% clay, 5min)

| Formulation solvent composition | | q (mol/g clay) | |
|---|---|---|---|
| % ethanol | % water | Sodium Laurate | Sodium Oleate |
| 25% | 75% | 5.395731E-05 | 5.840883E-05 |
| 35% | 65% | 5.894527E-05 | 5.466552E-05 |
| 50% | 50% | 7.069516E-05 | 5.650897E-05 |

We did not observe significant differences between laurate and oleate under the same experimental conditions, supporting the hypothesis that the structure of the hydrophobic tail of the FFA is not playing a significant role. This result is particularly relevant for real life applications, when complex mixtures of FFA would be produced at the skin by the metabolic activity of bacteria. The similarities observed between the saturated and unsaturated FFA studied here suggests a broad spectrum of FFA adsorption capacity of the clay, combined with a specific targeting of pH conditions compatible with damaged skin.

**Interference of citrate pH buffer on the adsorption capacity of the clay**

The ability of the clay to adsorb citrate buffer was evaluated using the protocol described for pH-metric titrations with the only variation that the pH of the clay solution was neutralised before use. Two sets of solutions were studied, one set containing the original buffer without contact with the clay and the other one having 5min of contact with the absorbent material. The buffer used was $10^{-2}$M citrate (pH=5) which is approved and commonly used additive to water based skin formulations. Comparison between these two data sets allows the determination of the amount of buffer adsorbed on the clay.

Conventional water based products commonly employ relatively high concentrations of pH buffers. The pH buffer would affect the pH responsivity of such systems, since no pH distinction between damaged and healthy skin is possible, i.e. the buffer will maintain the pH within the formulation irrespectively of the initial pH of the skin.

| | Initial pH | Final pH (after titration) | Titre (ml) | % Buffer adsorbed |
|---|---|---|---|---|
| Citrate buffer | 5.76 | 4.00 | 14.04 | - |
| Citrate buffer and clay | 5.11 | 4.00 | 9.76 | 30% |

It can be seen that the presence of clay reduces the amount of nitric acid required for the titration, indicating that the buffer has been adsorbed onto the clay.

Considering that citrate molecules contain carboxylic groups, we can infer that the large excess of buffer required to maintain the pH in water based formulations will interfere with the adsorption of the FFA that are the target of our technology. Therefore, the need of using ethanol rich formulations is essential to enhance both the adsorption and pH responsivity of our formulation.

**Kinetics of adsorption of FFA salts on the absorbent material.**

The kinetics of FFA adsorption was evaluated following protocols similar to the ones used for the fixed mass isotherms. The adsorption of FFA on the selected clay montmorillonite K10 was investigated using different solvent conditions. The clay Montmorillonite K10 was purchased from Sigma Aldrich and was used without any further treatment, unless otherwise specified. Two model fatty acid salts were investigated (i.e. sodium laurate and sodium oleate, Sigma Aldrich). For the determination of the absorption, 0.6g of clay samples were placed in 50ml falcon tubes and 20ml of the fatty salt solutions ($2 \times 10^{-3}$M) in different solvent mixtures from 55% to 0% ethanol in water were added, stirring vigorously to lead a cloudy suspension. Three different equilibration times were investigated, 30min, 15min, and 5min. For the 30min and 15min experiments, the samples were equilibrated for 25min and 10min respectively in a thermostat bath, and then subsequently centrifuged in a thermostated pre-heated centrifuge (T=37°C) for 5min to sediment the suspended clay. For the 5min experiments, the samples were immediately centrifuged for 5min in a thermostated pre-heated centrifuge (T=37°C) to sediment the suspended clay. After centrifugation, aliquots of 5ml were immediately taken from the supernatant on each sample to perform the potentiometric titration method described above. All the experiments were carried out in triplicate.

Considering the low effect of ethanol content on the adsorption of FFA on the clay, we selected a solvent

composition close to the minimum ethanol content compatible with skin formulations (20%). The clay content in these experiments was set to 3%.

The data obtained for the two model FFA salts used suggests that at contact times as short as 5minutes, the adsorption kinetics is completed, suggesting that the formulation is compatible with a cleansing type product. Times below 5 minutes were not experimentally accessible due to the centrifugation step required in order to separate the suspended clay from the solution to be analysed.

**FFAs produced from microbial enzymatic lipase activity under physiological conditions mimicking damages skin conditions**

**Lipase Assay**

The EnzChek lipase substrate (Roar Biomedical) was stored in DMSO at -20°C. The EnzChek lipase substrate solution was freshly prepared in 200 mM Tris-HCl, pH 7.5 and 0.0125% Zwittergent (Sigma Aldrich, UK) for each use, although this solution is stable if left at room temperature for at least 10 days. The uncatalyzed hydrolysis rate of the EnzChek lipase substrate was approximately 0.1 pmol $min^{-1}$. A total of 5 μl of model microbial lipoprotein lipase (LPL) from a bacterial species was used in the assay (Sigma Aldrich Ltd., UK). A typical assay was carried out at room temperature in a black 96-well plate (Thermo Fisher Scientific, UK) in the presence of freshly prepared 10 mM Tris-HCl, pH 7.5 in a total volume of 200 μl. The effect of variant concentrations of ethanol on biological LPL activity were tested in the buffer, replacing a set volume of with an identical volume of EtOH to the following concentrations: 0%, 5%, 10%, 20%, 30%. The Perkin Elmer Victor 3 1420 microplate reader and SoftMax Pro 5 (software) from MDS Analytics Technologies were used for fluorescent measurements. The maximum excitation/emission wavelengths were determined to be 482 nm/515 nm with a 495 nm filter cutoff, 0.1s mix. Fluorescence was measured at 1, 4 and 4.5 hr time points.

The use of a pH buffer in this experiment may appear inconsistent considering that pH buffers can interfere with the responsiveness of the formulation. However there is no contradiction. The relevance of avoiding pH buffers is related to the responsivity of the clay FFA adsorption *in vivo* (i.e. in real working conditions on the skin). Using a pH buffer on the skin will hinder the selective response by maintaining the pH of the formulation. In the experiments described in this specific section, the responsivity of the system was not evaluated, the pH was fixed at the "damaged skin" conditions, and only evaluated the ability of the formulation to adsorb FFA produced by microbial enzymatic lipase activity under physiological conditions mimicking damages skin conditions. Additionally, among different buffers, Tris was selected since it does not contain carboxylic acids.

**Adsorbent formulation**

After 4 hr incubation of the triglyceride model with the lipase enzyme in 200 μl, all 200 μl of substrate and LPL treated buffer were added to 1.5 ml Eppendorf tubes. To this, 100 μl of adsorbent formulation was added at either 15% or 25% concentrations. The reaction mix was incubated at RT for 10 mins before undergoing centrifugation at 14,000 x g for 20 mins. After centrifugation, the supernatant was collected and fluorescence measured using the parameters described above.

**Experimental results**

In order to quantify the removal of FFAs by absorbent clay formulations of differing concentrations, the relative fluorescence of each sample was measured after incubation with the 15% and 25% clay adsorbent formulations, or an equal volume of 30% ethanol. From this data, an average percentage of the total remaining in FFA was calculated by comparing the non-clay treated (30% ethanol) and formulation treated LPL-digested model triglyceride.

Figure 2 shows the quantification of adsorbent clay removal of FFA created by microbial lipase digestion of a model triglyceride. The data are shown as % of untreated control sample (clay replaced with ethanol). Panel A represents data from the use of the 15% clay formulation. Panel B represents data from the use of the 25% clay formulation. All readings are shown as % of remaining FFA, measured by comparing differences of relative fluorescence (above background) of adsorbent treated and non-adsorbent treated samples. Error bars represent St. Dev. (n=3).

The results demonstrate that the level of FFAs significantly decreased in contact with the adsorbent formulations for all the concentrations of ethanol, compared to samples not treated with the clay formulation.

The data also shows that no significant differences were observed in the remaining FFA (%) after treatment, across a range of ethanol concentration (0-30%). This indicates that the variation of ethanol concentration in the Tris-HCl buffer has no effect on FFA adsorption by the clay. Furthermore, there was also no significant variation between treatments with the 15% or 25% adsorbent clay formulations indicating that both formulations are able to adsorb FFAs to a similar extent under physiological pH conditions mimicking damaged skin. Both formulations on average resulted

in a decrease of ~80% of the FFAs released by the enzymatic activity of the bacterial lipase on the model triglyceride.

## Claims

1. A cosmetic composition comprising:

   2 to 60wt% clay;
   2 to 60wt% preservative; and
   20 to 96wt% water;

   wherein the cosmetic composition comprises no more than 1% carboxylic acid compounds and the ratio of clay to carboxylic acid compounds is at least 90wt% clay: no more than 10wt% carboxylic acid compounds.

2. The composition of claim 1, which has a pH of at least 6.5.

3. The composition of claim 1 or claim 2, comprising no more than 3wt% pH buffer.

4. The composition of any one of the preceding claims, wherein the clay comprises a 2:1 clay, the 2:1 clay having one octahedral silicate sheet between two tetrahedral silicate sheets.

5. The composition of any one of the preceding claims, wherein (i) the clay comprises montmorillonite; and/or (ii) the preservative comprises alcohol.

6. The composition of any one of the preceding claims, wherein the composition has a pH of from 6.5 to 7.5 and no pH buffer is present.

7. The composition of any one of the preceding claims comprising

   4 to 40wt% clay;
   4 to 40wt% preservative; and
   20 to 92wt% water

   wherein the cosmetic composition comprises no more than 0.5wt% carboxylic acid compounds and the ratio of clay to carboxylic acid compounds is at least 95wt% clay: no more than 5wt% carboxylic acid compounds.

8. The composition of any one of the preceding claims, the composition consisting of montmorillonite, ethanol and water.

9. The composition of any one of claims 1 to 7, which comprises 0.1 to 5wt% hydroxyalkyl cellulose.

10. Use of the cosmetic composition of any one of the preceding claims for selective removal of free fatty acids (FFAs) from the skin.

11. The composition of any one of claims 1 to 9 for use as a medicament.

12. The composition of any one of claims 1 to 9 for use in the treatment of a skin condition.

13. A method of cosmetic treatment of a skin condition comprising the step of applying the cosmetic composition of any one of claims 1 to 9 onto the skin of a subject afflicted with the skin condition or at risk of being afflicted with the skin condition.

14. The composition for use of claim 12 or the method of claim 13, wherein the skin condition is acne vulgaris.

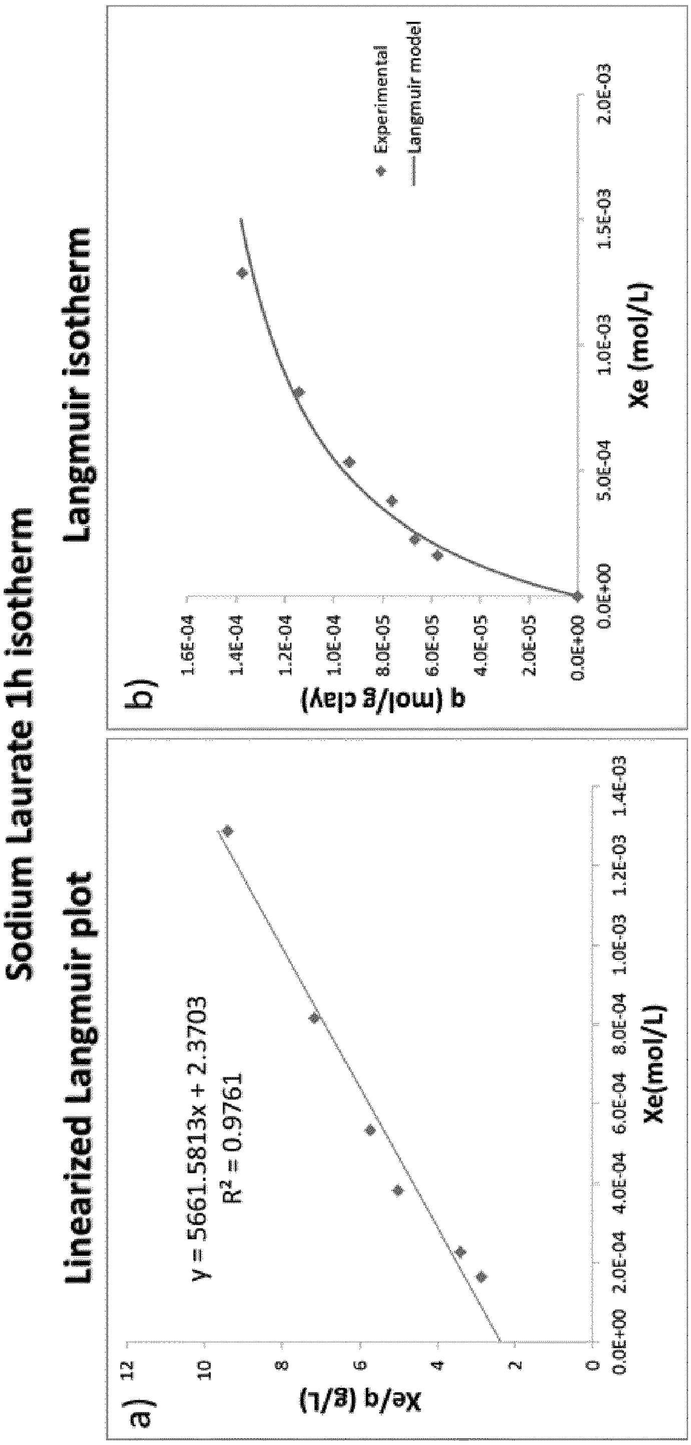
Sodium Laurate 1h isotherm
Linearized Langmuir plot
a)
y = 5661.5813x + 2.3703
R² = 0.9761
Xe/q (g/L)
Xe(mol/L)
Langmuir isotherm
b)
q (mol/g clay)
Xe (mol/L)
Experimental
Langmuir model

Fig 1

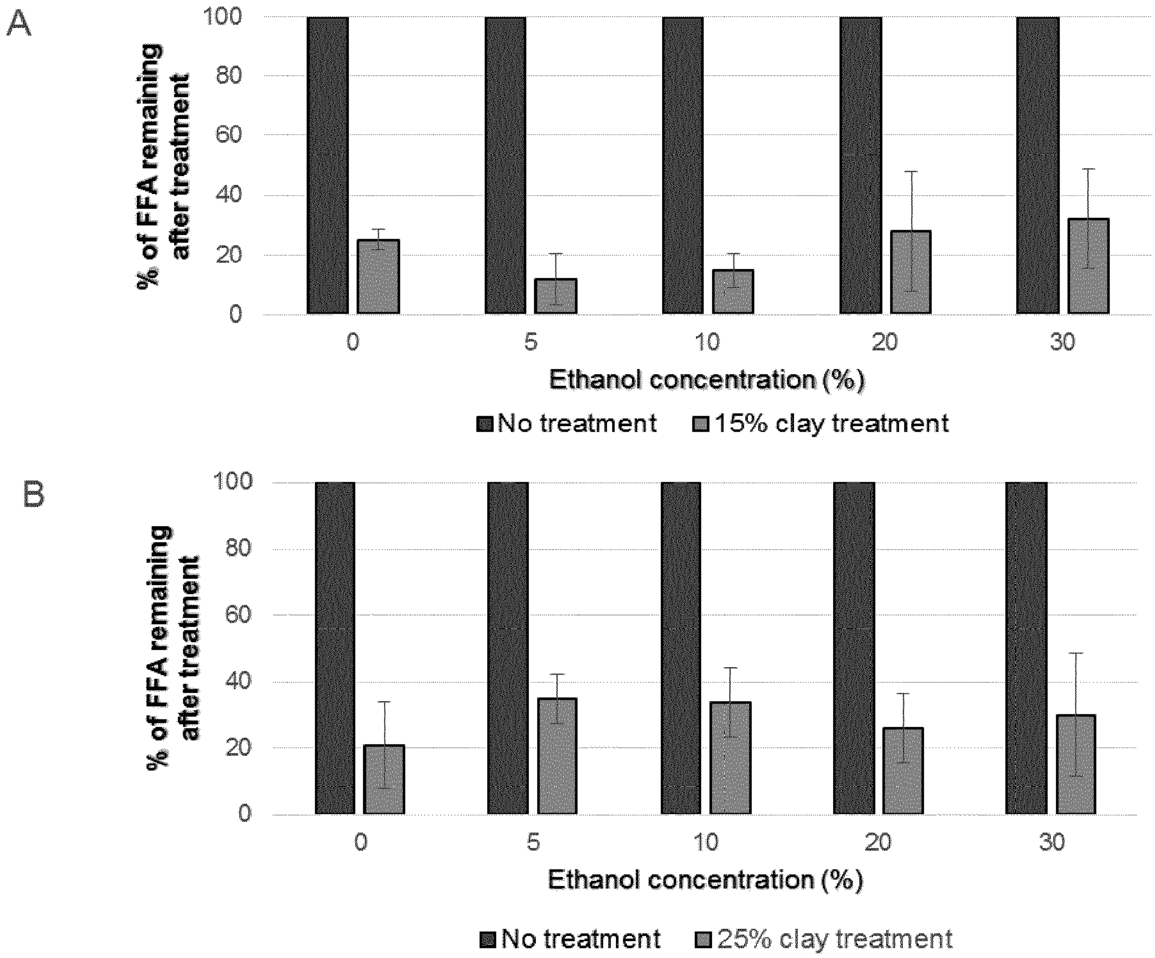
A
100
80
60
40
20
0
% of FFA remaining after treatment
0
5
10
20
30
Ethanol concentration (%)
No treatment
15% clay treatment
B
100
80
60
40
20
0
% of FFA remaining after treatment
0
5
10
20
30
Ethanol concentration (%)
No treatment
25% clay treatment

**Fig 2**

Europäisches Patentamt
European Patent Office
Office européen des brevets

# EUROPEAN SEARCH REPORT

Application Number
EP 18 02 0186

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 640 932 A (FONG JOHN [US] ET AL) 3 February 1987 (1987-02-03) * examples * | 1-7,9-14 | INV. A61K8/26 A61K8/34 A61K8/73 A61Q19/10 A61P17/10 |
| X | YUJI KIMURA ET AL: "Clay-Alcohol-Water Dispersions: Anomalous Viscosity Changes Due to Network Formation of Clay Nanosheets Induced by Alcohol Clustering", LANGMUIR, vol. 33, no. 19, 16 May 2017 (2017-05-16), pages 4758-4768, XP55507753, US ISSN: 0743-7463, DOI: 10.1021/acs.langmuir.7b00764 *preparation of ninary and ternary dispersion on p. 4759* | 8 | |
| X | US 4 388 301 A (KLEIN ROBERT W) 14 June 1983 (1983-06-14) * examples * | 1-7, 10-14 | |

TECHNICAL FIELDS SEARCHED (IPC)

A61K
A61Q
A61P

The present search report has been drawn up for all claims

3

| Place of search | Date of completion of the search | Examiner |
|---|---|---|
| Munich | 19 September 2018 | Rinkel, Bert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 02 0186

This annex lists the patent family members relating to the patent documents cited in the above-mentioned European search report. The members are as contained in the European Patent Office EDP file on
The European Patent Office is in no way liable for these particulars which are merely given for the purpose of information.

19-09-2018

| Patent document cited in search report | Publication date | Patent family member(s) | Publication date |
|---|---|---|---|
| US 4640932 A | 03-02-1987 | AT 71294 T | 15-01-1992 |
| | | AU 581590 B2 | 23-02-1989 |
| | | CA 1261757 A | 26-09-1989 |
| | | DK 548986 A | 17-11-1986 |
| | | EP 0215108 A1 | 25-03-1987 |
| | | US 4640932 A | 03-02-1987 |
| | | WO 8605394 A1 | 25-09-1986 |
| US 4388301 A | 14-06-1983 | AR 227559 A1 | 15-11-1982 |
| | | AU 548845 B2 | 02-01-1986 |
| | | BE 890648 A | 01-02-1982 |
| | | BR 8109030 A | 10-05-1983 |
| | | CA 1185530 A | 16-04-1985 |
| | | CH 657528 A5 | 15-09-1986 |
| | | DE 3152893 A1 | 16-06-1983 |
| | | DK 60083 A | 11-02-1983 |
| | | EP 0081492 A1 | 22-06-1983 |
| | | ES 8301624 A1 | 01-01-1983 |
| | | GB 2110932 A | 29-06-1983 |
| | | IE 51641 B1 | 21-01-1987 |
| | | IT 1144832 B | 29-10-1986 |
| | | JP S58500897 A | 02-06-1983 |
| | | NL 8120306 A | 02-05-1983 |
| | | NZ 198575 A | 30-04-1985 |
| | | PH 20226 A | 23-10-1986 |
| | | US 4388301 A | 14-06-1983 |
| | | WO 8204393 A1 | 23-12-1982 |
| | | ZA 8107536 B | 27-10-1982 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LWIN et al.** *Clinical and Experimental Dermatology,* 2014, vol. 39, 162-167